# DEMANDE DE BREVET EUROPEEN

(11) **EP 0 624 800 A1**
(43) Date de publication de la demande: **17.11.1994**
(21) Numéro de dépôt: 94401028.9
(22) Date de dépôt: 10.05.1994
(51) Int. Cl.: G01N 33/543, B01D 61/00, B01D 61/14

(54) **Détermination de substances dans un milieu liquide par filtration tangentielle**

(30) Priorité: 13.05.1993 FR 9305752
(71) Demandeur: ETAT FRANCAIS, Représenté par le Délégué Général, pour l'Armement, Paris 7ème (FR)
(72) Inventeur: Therasse, Joel, F-91710 Vert-le-Petit (FR); Ricaud, Nicole, F-91810 Vert-le-Grand (FR); Binder, Patrice, F-91710 Vert-le-Petit (FR)

(57) **Abrégé**

L'invention concerne un procédé et un dispositif pour la détermination qualitative et quantitative de substances dans un milieu liquide par filtration tangentielle.

Le procédé de filtration est caractérisé en ce qu' on utilise une membrane (4) rendue spécifique par immobilisation d'au moins un ligand spécifique de chacune des substances recherchées, on fait circuler le milieu liquide (5) à grande vitesse tangentiellement à la surface de la membrane, on maintient une légère surpression au-dessus de la membrane de filtration.

Le dispositif comprend une cellule (16) de détection et/ou de mesure, des moyens (19) pour la circulation des liquides et des moyens assurant l'étanchéité. La cellule comprend au moins une enceinte, chaque enceinte ayant un espace interne pour le passage du liquide et un support perforé (3) portant la membrane microporeuse (4,8) sur laquelle des ligands sont immobilisés.

## Description

La présente invention concerne la détermination qualitative et quantitative de substances dans un milieu liquide par filtration tangentielle.

Il est connu de détecter des substances telles que des substances biologiques, des micro-organismes ou des substances chimiques présentes dans des milieux liquides à l'aide de réactifs doués d'affinité pour lesdites substances. On peut citer par exemple les procédés fondés sur la réaction antigène - anticorps. Généralement ces procédés nécessitent des temps d'incubation importants qui résultent des lois physico-chimiques des solutés en présence.

Pour la détection de substances biologiques, une amélioration a été apportée par le procédé dit ELIFA (Enzyme Linked Immuno-Filtration Assay) fondé sur le principe de la mise en contact des différents réactifs nécessaires à l'identification de la substance biologique par une filtration active à travers une membrane sélective sur laquelle sont immobilisés les différents réactifs nécessaires (Pinon J. M., et al, J. Immunol. Methods 1985, 77, 15-23 ; H. Dupont, et al, J. Immunol. Methods 1990, 128, 287-291). Ce procédé permet de réduire les temps d'incubation, mais son efficacité est limitée par les problèmes de colmatage de la membrane et par le fait que le liquide à analyser ne peut l'être que par petites fractions.

On connaît en outre les procédés d'extraction d'une substance contenue dans un liquide par une filtration tangentielle. La mise en oeuvre d'une filtration tangentielle nécessite la présence d'une surpression importante au-dessus de la membrane de filtration afin de faire percoler une partie du liquide.

La présente invention a pour but de permettre la détermination qualitative et quantitative d'une ou de plusieurs substances contenues dans un milieu liquide, par un procédé rapide, efficace et économique, qui ne présente pas les inconvénients des procédés de l'art antérieur précités.

A cet effet, la présente invention a pour objet un procédé de détermination qualitative et/ou quantitative d'une ou de plusieurs substances susceptibles d'être présentes dans un milieu liquide, ainsi qu'un dispositif pour la mise en oeuvre du procédé.

Le procédé selon l'invention utilise une filtration ayant lieu dans une cellule de détection et/ou de mesure, et est caractérisé en ce qu'il comprend les étapes successives suivantes :
a - utilisation d'une membrane activée ou rendue spécifique par immobilisation dans la structure microporeuse de la membrane d'une quantité connue d'au moins un premier ligand spécifique de chaque substance à rechercher et à quantifier.
b - mise en contact du milieu liquide renfermant la ou les substances avec la membrane de manière répétée par une circulation du liquide tangentiellement à la surface de la membrane jusqu'à épuisement de ou des substances, tout en maintenant une légère surpression au-dessus de la membrane de filtration.
c - mise en contact de la membrane avec un liquide contenant au moins un second ligand spécifique par substance à rechercher, ce ou ces seconds ligands étant directement ou indirectement détectable.
d - mesure de la quantité de chaque second ligand fixé spécifiquement sur la ou les substances à isoler et à quantifier retenues sur la membrane.
e - détermination à partir des résultats de l'étape d), de la présence ou de l'absence de ou des substances recherchées, ainsi que de la quantité de chaque substance présente.

Dans le procédé de l'invention, le liquide circule sur la surface de la membrane à grande vitesse, une partie dudit liquide percole à travers la membrane microporeuse, l'autre partie reste au-dessus de la membrane et est remise en circulation jusqu'à épuisement.

La circulation tangentielle milieu liquide à la surface de la membrane permet d'assurer un nettoyage de la membrane et d'éviter le colmatage habituellement observé lorsque le principe de l'immunofiltration est appliqué à des milieux complexes contenant des particules solides. En outre, l'utilisation d'une membrane activée rendue spécifique exerce un effet moteur pour la percolation du liquide qui doit traverser la membrane. La surpression au-dessus du liquide peut ainsi être maintenue à une valeur faible.

Le procédé de la présente invention peut être mis en oeuvre pour la détermination qualitative et quantitative de toute substance présente dans un milieu liquide, dès lors qu'il existe un élément qui présente une affinité avec ladite substance et qui peut être immobilisé sur la structure microporeuse de la membrane. La substance peut être une substance chimique, une substance biologique telle qu'une protéine, ou un microorganisme tel qu'un virus, une bactérie, une toxine fongique ou marine.

Une catégorie particulière de ligands est fournie par les anticorps.

Le procédé de l'invention peut ainsi être mis en oeuvre pour toutes les déterminations par une méthode immunologique, et notamment pour tous les couples récepteur biologique/ligand correspondant (ou agoniste ou antagoniste). Suivant les cas, l'un ou l'autre élément du couple pourra être immobilisé dans la membrane microporeuse. A titre d'exemple, on peut citer les couples marqueur de cellules/cellules, marqueur de microorganisme/microorganisme, HcG/récepteur tissulaire, anticorps/antigène, haptène/anticorps, ADNc/ADN, poly dT/ARNm eucaryote, enzyme/substrat, enzyme/inhibiteur d'enzyme, lectine/glycoconjugué.

Un exemple particulièrement intéressant d'application du procédé de l'invention est constitué par l'hybridation de sondes nucléiques entre un oligonucléotide immobilisé sur la membrane et l'acide nucléique portant la séquence complémentaire. De même que dans le cas de systèmes immunologiques, le signal est obtenu par une deuxième sonde nucléique utilisée dans la phase liquide.

Le procédé de l'invention peut également être mis en oeuvre pour traiter des liquides contenant des substances chimiques, dès lors que pour une substance chimique donnée, il existe un ligand qui présente une affinité avec ladite substance et qui peut être immobilisé dans la membrane microporeuse. A titre d'exemple, on peut citer les couples cation métallique/ligand chélatant, anticorps anti-haptène/molécule de faible poids moléculaire, enzyme/substrat.

La sensibilité du système dépend des dimensions de la cellule de filtration, de la surface spécifique de la membrane, de la quantité de ligand immobilisé dans la membrane et des temps de réaction imposés.

Le liquide traité peut être toute solution tampon compatible avec le couple élément à détecter-élément détecteur utilisé. Les interactions non spécifiques par échange d'ions ou par interactions hydrophobes peuvent être combattues par des tampons de force ionique appropriée contenant éventuellement des détergents ou toute autre substance utilisée classiquement.

Le procédé de l'invention permet de d'identifier au cours d'une même opération, une ou plusieurs substances présentes dans le liquide à traiter. Lorsque l'on veut identifier simultanément plusieurs substances présentes dans un liquide, l'on utilise pour la filtration, une membrane comportant autant de zones que de substances à identifier, chaque zone étant activée ou rendue spécifique par un premier ligand spécifique de chaque substance et éventuellement une ou plusieurs zones témoins fixant le niveau de bruit de fond et des zones portant des témoins positifs.

Dans un mode de réalisation particulier du procédé de la présente invention, après avoir fait circuler jusqu'à épuisement le milieu liquide à analyser sur la membrane microporeuse portant un premier type de ligand spécifique d'une substance à identifier, on fait circuler sur la membrane un liquide contenant un second ligand spécifique de ladite substance à identifier, ce second ligand étant porteur d'une activité détectable par un moyen physique, chimique ou biologique. Dans une variante, on utilise le second ligand couplé à une enzyme et l'on effectue ensuite à l'aide d'un substrat précipitant, la mesure de l'activité de l'enzyme retenue par le second ligand. Dans une autre variante, on fixe le second ligand sur de petites particules marquées par une substance détectable par un moyen physique, chimique ou biologique et capables de se maintenir en suspension dans le milieu liquide. La substance détectable est avantageusement constituée par un colorant.

Le procédé de la présente invention permet non seulement de détecter et d'identifier une ou plusieurs substances présentes dans le liquide à traiter, mais il permet également une détermination quantitative desdites substances. A cet effet, l'on immobilise une quantité connue de ligand sur la membrane et l'on peut effectuer soit une détermination par capture directe du ligand, soit par compétition entre le ligand en solution et un ligand compétiteur immobilisé sur la membrane au préalable.

Lorsque l'on souhaite effectuer une détermination quantitative d'une substance biologique dont on connaît la présence dans le milieu liquide, il est particulièrement intéressant de mélanger ladite substance avec une quantité connue de la même substance fixée sur une protéine enzymatique ou sur de petites particules individuelles marquées par une substance détectable par un moyen physique, chimique ou biologique, par exemple par un colorant, avant de mettre ladite substance à doser en présence du ligand immobilisé sur la membrane.

La présente invention a également pour objet un dispositif pour la mise en oeuvre du procédé. Le dispositif de la présente invention comprend essentiellement une cellule de détection et/ou de mesure et un module hydraulique pour la circulation des liquides à analyser et des moyens pour assurer l'étanchéité de l'ensemble. Il est caractérisé en ce que la cellule de mesure comprend une ou plusieurs enceintes, chaque enceinte comportant un espace interne pour la circulation de l'échantillon liquide et un support perforé pour une membrane microporeuse sur laquelle des ligands sont immobilisés. Le dispositif de l'invention permet de traiter des volumes de liquides très variés, du fait que l'échantillon liquide à traiter est mis en circulation à la surface de la membrane.

La présente invention est expliquée plus en détail par référence aux dessins annexés qui sont donnés à titre d'illustration. Il est bien entendu que l'invention n'est pas limitée au mode de réalisation représenté.

Dans les dessins annexés :
- la figure 1 représente une vue schématique éclatée d'une enceinte de filtration, destinée à illustrer le principe du procédé de l'invention.
- la figure 2 représente une vue éclatée d'une cellule pour la mise en oeuvre du procédé de l'invention.
- la figure 3 représente un plan schématique d'une installation complète pour la mise en oeuvre du procédé de l'invention.

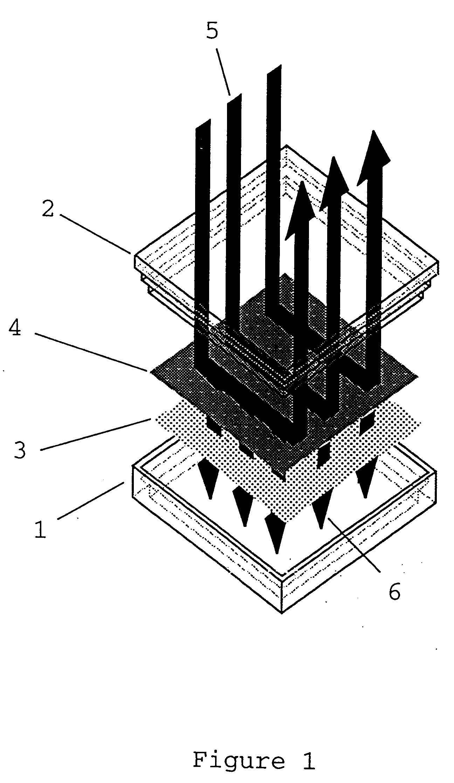

L'enceinte de filtration représentée sous forme éclatée sur la figure 1 comprend un bloc inférieur 1, un bloc 2 supérieur, les deux étant par exemple en Plexiglas®, une grille 3, constituée par exemple par du Téflon®, ladite grille 3 supportant une membrane 4 sur laquelle sont immobilisés des ligands. Le flux 5 de liquide à analyser circule de manière tangentielle sur la surface de la membrane 4. Une partie 6 du flux liquide percole à travers la membrane 4 sous l'effet d'une surpression exercée sur le liquide constituant le flux 5 et est recueillie dans le bloc inférieur 1. L'essentiel de la partie du flux 5 qui n'a pas percolé est recyclé.

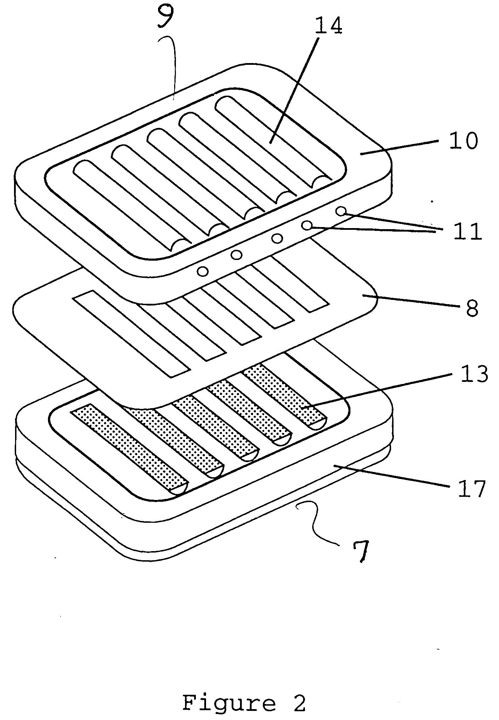

La figure 2 représente un mode de réalisation particulier d'une cellule de filtration utilisable pour la mise en oeuvre du procédé de l'invention. Elle comporte une partie inférieure 7, une membrane 8 et une partie supérieure 9. La partie inférieure 7 sert de support à la membrane 8 et permet de collecter les effluents. La partie supérieure 9 comporte des moyens 11 pour l'introduction du milieu liquide à analyser ou de divers réactifs et des moyens 10 (non représentés sur la figure 2) pour leur sortie. La face supérieure de la partie inférieure 7 est munie d'au moins une cavité 13 et la face inférieure de la partie supérieure 10 d'au moins une cavité 14. Chaque face a le même nombre de cavités. Les cavités de l'une des faces ont la même forme que les cavités de l'autre face et elles se trouvent exactement en regard les unes des autres, afin de constituer des compartiments dans lesquels un liquide peut circuler.

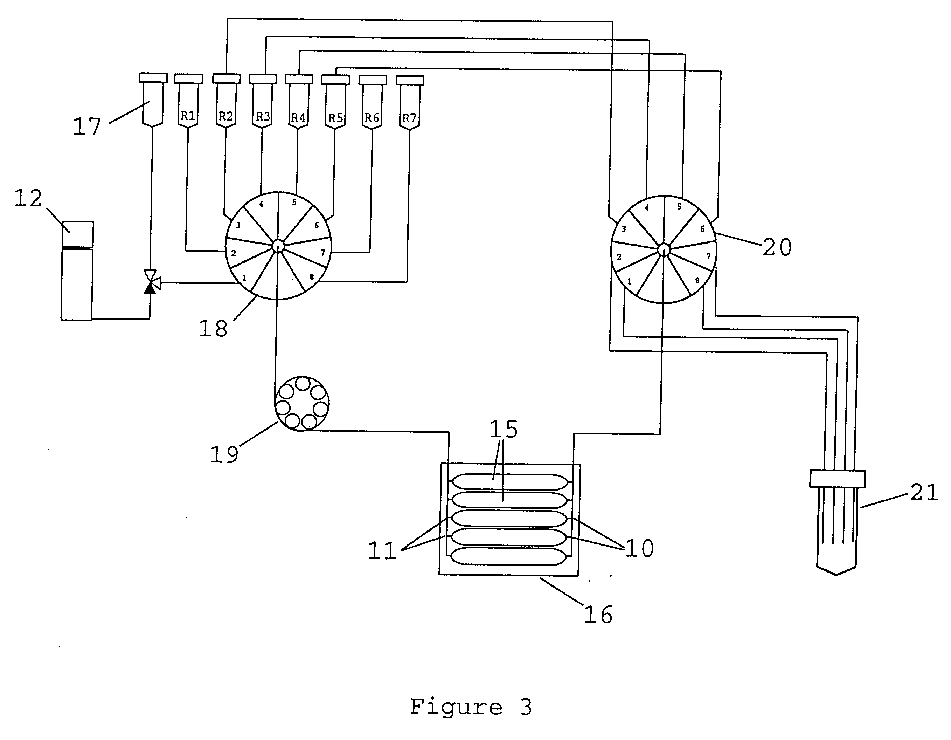

La figure 3 représente une vue d'ensemble d'une installation pour la mise en oeuvre du procédé de la présente invention. Cette installation comprend une cellule de filtration 16 constituée par plusieurs compartiments 15 comportant des moyens d'introduction 11 et des moyens de sortie de liquides 10, un réservoir 17 pour l'introduction manuelle de l'échantillon à analyser, un système 12 pour un éventuel prélèvement direct de l'échantillon à analyser et des réservoirs R1 à R7 (ce nombre de réservoirs étant donné à titre d'exemple) contenant différents réactifs, une vanne 18 multivoies (huit voies dans le cas présent) et une pompe péristaltique 19 reliant le réservoir à échantillon 17 et les réservoirs à réactifs R1 à R7 à la cellule de filtration 16, un boîtier de décontamination 21, une seconde vanne multivoies 20 reliant la cellule de filtration 16 aux différents réservoirs et au boîtier de décontamination. La cellule comprend en outre des moyens, non représentés, pour extraire de la cellule de filtration les fractions de liquide qui ont percolé à travers la membrane dans les différents compartiments, et pour les diriger vers le boîtier de décontamination 21.

La présente invention est illustrée par l'exemple suivant, étant entendu que cet exemple ne limite pas la portée de l'invention.

### EXEMPLE

Une installation conforme à celle de la figure 3 a été utilisée pour mettre en évidence la présence d'une toxine, l'entérotoxine B de Staphylococcus aureus dans un échantillon liquide à l'aide d'une membrane activée au préalable avec un anticorps spécifique.

L'échantillon à analyser contenant l'entérotoxine B, placé dans le réservoir 17, a été mis en contact avec la membrane activée par circulation tangentielle à un débit de 5 ml/min pendant 5 min. Une très faible fraction de la solution a percolé à travers la membrane et a été dirigée vers le réservoir 21.

La membrane ayant retenu sélectivement l'entérotoxine B a ensuite été lavée par circulation pendant 1 min d'une solution de lavage [tampon Tris 20 mM pH 7,2 contenant 0,9% de chlorure de sodium, Tris désignant un tris(hydroxyméthyl) aminométhane] provenant par exemple du réservoir R1.

Une solution d'un second anticorps anti-entérotoxine B couplé à une enzyme, la peroxydase du Raifort, contenue dans l'un des réservoirs R2 à R5, a ensuite été mise en circulation tangentielle avec un débit de 5 ml/min pendant 2 min à la surface de la membrane. Puis la membrane a été lavée pendant 1 min par circulation sur sa surface de la solution de lavage contenue dans le réservoir R1.

L'activité enzymatique de la peroxydase du Raifort retenue sur la membrane par l'intermédiaire de l'entérotoxine B capturée par le premier anticorps anti-entérotoxine B qui a servi à activer la membrane a été révélée en remplissant les compartiments 15 avec une solution de α-chloronaphtol dans un tampon Tris 20 mM pH 7,2 contenant 0,9% de chlorure de sodium. Après 3 min d'incubation statique de cette solution, la pompe 19 étant à l'arrêt, la présence d'enzyme, et par conséquent d'entérotoxine B, a été quantifiée par l'apparition d'une coloration précipitante sur la membrane plus ou moins intense de couleur brune.

## Revendications

**1 -** Procédé de détermination qualitative et quantitative d'une ou plusieurs substances contenues dans un milieu liquide au moyen d'une filtration ayant lieu dans une cellule de détection et/ou de mesure, caractérisé en ce qu'il comprend les étapes successives suivantes :
a - utilisation d'une membrane activée ou rendue spécifique par immobilisation dans la structure microporeuse de la membrane d'une quantité connue d'au moins un "premier ligand" spécifique de chaque substance à rechercher et à quantifier,
b - mise en contact du milieu liquide renfermant la ou les substances avec la membrane de manière répétée par une circulation du liquide tangentiellement à la surface de la membrane jusqu'à épuisement de ou des substances, tout en maintenant une légère surpression au-dessus de la membrane de filtration,
c - mise en contact de la membrane avec un liquide contenant au moins un "second ligand" spécifique par substance à rechercher, ce ou ces "seconds ligands" étant directement ou indirectement détectable,
d - mesure de la quantité de chaque "second ligand" fixé spécifiquement sur la ou les substances à isoler et à quantifier retenues sur la membrane,
e - détermination à partir des résultats de l'étape d), de la présence ou de l'absence de ou des substances recherchées, ainsi que de la quantité de chaque substance présente.

**2 -** Procédé selon la revendication 1, caractérisé en ce que la membrane sur laquelle circule le milieu liquide comprend une ou plusieurs zones, chaque zone étant activée ou rendue spécifique par un "premier ligand" spécifique de chaque substance à détecter et à quantifier.

**3 -** Procédé selon l'une des revendications 1 ou 2, caractérisé en ce que le ou les "premiers ligands" immobilisés dans la structure microporeuse sont spécifiques de substances chimiques.

**4 -** Procédé selon l'une des revendications 1 ou 2, caractérisé en ce que le ou les "premiers ligands" immobilisés dans la structure microporeuse sont spécifiques de substances biologiques.

**5 -** Procédé selon l'une des revendications 1 ou 2, caractérisé en ce que le ou les "premiers ligands" immobilisés dans la structure microporeuse sont spécifiques de microorganismes.

**6 -** Procédé selon l'une des revendications 1 ou 2, caractérisé en ce que le ou les "premiers ligands" immobilisés dans la structure microporeuse sont du type anticorps.

**7 -** Procédé selon la revendication 1, caractérisé en ce que la détection du ou des "seconds ligands" est réalisée par un moyen chimique, physique ou biologique.

**8 -** Procédé selon l'une des revendications 1 ou 7, caractérisé en ce que le ou les "seconds ligands" sont couplés à un ou à des enzymes, et que l'on mesure l'activité de ou des enzymes afin de détecter et de quantifier la ou les substances recherchées fixant spécifiquement ce ou ces "seconds ligands".

**9 -** Procédé selon l'une des revendications 1 ou 7, caractérisé en ce que le ou les "seconds ligands" sont couplés à de petites particules marquées par une substance détectable et capable de se maintenir en suspension dans le milieu liquide.

**10 -** Procédé selon l'une des revendications 1, 7 ou 9, caractérisé en ce que la substance détectable est un colorant.

**11 -** Procédé selon la revendication 1, caractérisé en ce que la détermination quantitative de ou des substances est faite par capture directe de la ou des substances par le ou les ligands fixés à la membrane et spécifiques de cette ou ces substances.

**12 -** Procédé selon la revendication 1, caractérisé en ce que la détermination quantitative de la ou des substances est faite par compétition entre un ou plusieurs ligands spécifiques libres en solution et le ou les ligands de même spécificité immobilisés sur la membrane.

**13 -** Procédé selon la revendication 1, caractérisé en ce que le ou les "seconds ligands" de l'étape c) sont remplacés par des quantités connues de la ou des substances à analyser couplées à des protéines enzymatiques ou à des petites particules détectables par un moyen chimique, physique ou biologique, ces quantités connues de substances "marquées" étant introduites dans la cellule de filtration en même temps que le milieu liquide à l'étape b).

**14 -** Procédé selon la revendication 1, caractérisé en ce qu'il est mis en oeuvre en immobilisant sur la membrane l'un des deux éléments constituant l'un ou plusieurs des couples choisis dans le groupe suivant : marqueur de cellules/cellules, marqueur de microorganismes/microorganismes, HcG/récepteur tissulaire, anticorps/antigène, haptène/anticorps, ADNc/ADN, poly dT/ARNm eucaryote, enzyme/substrat, enzyme/inhibiteur d'enzyme, lectine/glycoconjugué.

**15 -** Dispositif pour la mise en oeuvre du procédé selon une quelconque des revendications 1 ou 14, comprenant une cellule de détection et/ou de mesure, un module hydraulique pour la circulation des liquides à analyser et des moyens pour assurer l'étanchéité du dispositif, caractérisé en ce que la cellule comprend une ou plusieurs enceintes, chaque enceinte comportant un espace interne pour le passage de l'échantillon liquide et un support perforé pour une membrane microporeuse sur laquelle des ligands sont immobilisés.
